# FASCICULE DE BREVET EUROPEEN

(11) **EP 4 574 055 B1**
(45) Date de publication et mention de la délivrance du brevet: **19.08.2026**
(21) Numéro de dépôt: 24218568.4
(22) Date de dépôt: 10.12.2024
(51) Int. Cl.: A61B 8/08, A61B 8/00

(54) **METHODE D'IMAGERIE ULTRASONORE POUR LA LOCALISATION OU LE DIMENSIONNEMENT PRECIS D'UN OBJET**
ULTRASCHALLABBILDUNGSVERFAHREN ZUR GENAUEN LOKALISIERUNG ODER DIMENSIONIERUNG EINES OBJEKTS
ULTRASONIC IMAGING METHOD FOR ACCURATE LOCALIZATION OR SIZING OF AN OBJECT

(30) Priorité: 18.12.2023 FR 2314348
(43) Date de publication de la demande: 25.06.2025
(73) Titulaire: Commissariat à l'Energie Atomique et aux Energies Alternatives, 75015 Paris (FR)
(72) Inventeur: BELHADJ, Djallel, 91191 GIF-SUR-YVETTE Cedex (FR); CHATILLON, Sylvain, 91191 GIF-SUR-YVETTE Cedex (FR)
(74) Mandataire: Atout PI Laplace

(56) Documents cités:
- JP-B2- 5 854 072
- US-A1- 2021 100 523

## Description

L'invention concerne le domaine de l'imagerie ultrasonore, en particulier l'imagerie médicale, et porte sur une méthode d'amélioration de la précision de telles mesures lorsqu'elles sont réalisées au moyen d'une sonde ultrasonore ayant un champ d'ouverture du champ ultrasonore émis qui présente une taille donnée.

L'invention s'applique notamment pour l'imagerie de vaisseaux sanguins, par exemples d'artères, en vue de déterminer précisément le diamètre de tels vaisseaux, mais elle n'est pas limitée à cette application et vise plus généralement l'amélioration de la précision de mesure pour l'imagerie ultrasonore de tous types d'objet dans le domaine médical ou le domaine du contrôle non destructif par ultrasons.

De façon générale et en particulier dans le domaine médical, l'imagerie ultrasonore est un outil permettant la surveillance, parfois en temps réel, précise de certains objets anatomiques tels que les vaisseaux sanguins. En particulier, elle permet de localiser et mesurer les dimensions de ces vaisseaux. Cependant la précision des mesures ultrasonores varie selon le type d'équipement utilisé.

Le développement des technologies à base d'ultrasons permet d'avoir des systèmes de surveillance compacts offrant une solution non invasive. Une surveillance temps réel portable entièrement automatisée permet de fournir, à la fois, une information pertinente sur la vie quotidienne ainsi que des données sur des durées plus grandes et ce dans des situations inaccessibles dans les services de soins avec des appareils fixes.

Cependant, les appareils d'imagerie ultrasonore portables sont limités en ressources et en consommation énergétique, cela impose un compromis sur la précision et les fréquences utilisées, ce qui limite à son tour l'exploitabilité des données collectées. Ces limitations en fréquence et en précision rendent moins précoce la découverte de maladies et limitent l'avancée et la compréhension des phénomènes d'intérêt, par exemple des maladies cardiovasculaires dans le domaine médical. Classiquement, les solutions existantes tendent à privilégier la précision au détriment de la consommation énergétique, du faible coût et de la simplicité des algorithmes.

Il existe donc un problème général consistant à améliorer la précision des systèmes d'imagerie ultrasonore sans augmenter leur consommation énergétique, leur coût, et sans besoin de modifier les équipements existants.

Dans le domaine de l'imagerie médicale, plusieurs méthodes existantes permettent de détecter et de caractériser des artères. On peut citer notamment les références [1] et [2] ainsi que les demandes de brevet WO206057233, US2021/100523A1 et JP5854072B2.

Toutes ces méthodes présentent l'inconvénient de souffrir d'une précision de mesure limitée car elles sont le plus souvent basées sur une méthode d'acquisition ultrasonore qui utilise un ensemble d'éléments pour générer un champ ultrasonore avec une ouverture ayant une taille qui dépend du nombre d'éléments. La précision des mesures dépend de différents paramètres en particulier la fréquence et la bande passante du signal. Mais elle dépend également de la dimension de la fenêtre active de la sonde, c'est-à-dire de la dimension totale de l'ensemble d'éléments actifs.

Plus la fenêtre active est petite, plus la précision de la mesure est élevée en terme de résolution. Cependant, une fenêtre active trop petite présente l'inconvénient de baisser le rapport signal à bruit car l'amplitude du signal est plus faible. C'est pour cette seconde raison que les sondes ultrasonores sont généralement basées sur des transducteurs multiéléments qui utilisent plusieurs éléments actifs pour synthétiser un faisceau ultrasonore afin de focaliser le faisceau par sommation cohérente des amplitudes des différents éléments.

L'invention est basée sur une solution algorithmique qui permet d'améliorer la résolution et la précision de la mesure sans modifier l'équipement d'acquisition. La méthode est basée sur la réalisation de plusieurs mesures successives en faisant varier le nombre d'éléments actifs ou la taille d'ouverture du champ ultrasonore généré puis en extrapolant la mesure qui aurait été obtenue avec une ouverture proche de zéro à partir des différents points de mesure réalisés.

L'invention a pour objet une méthode de caractérisation d'un objet au moyen d'une sonde ultrasonore, la méthode comprenant les étapes de :
- Réaliser plusieurs itérations des étapes de :
   i. Emettre un champ ultrasonore ayant une ouverture d'une taille donnée,
   ii. Mesurer un écho du champ ultrasonore après réflexion sur une zone d'intérêt d'un objet à imager,
   iii. A chaque nouvelle itération, modifier la taille de l'ouverture du champ ultrasonore,
- Pour au moins un point d'intérêt de la zone d'intérêt, relever , sur la mesure de l'écho, le temps de vol associé,
- Déterminer, à partir desdites mesures de temps de vol, un modèle de variation des temps de vol en fonction de la taille de l'ouverture du champ ultrasonore,
- Extrapoler le temps de vol du point d'intérêt pour une taille d'ouverture tendant vers 0 à partir dudit modèle.

Selon un aspect particulier de l'invention, la sonde ultrasonore comprend une pluralité d'éléments et la variation de la taille de l'ouverture du champ ultrasonore est obtenue en variant le nombre d'éléments actifs en réception.

Selon un aspect particulier de l'invention, le nombre d'éléments actifs en réception est un multiple de deux.

Selon un aspect particulier de l'invention, le nombre d'éléments actifs en émission est égal au nombre d'éléments actifs en réception ou au nombre maximal d'éléments que comprend la sonde.

Selon un aspect particulier de l'invention, l'objet à imager est un vaisseau sanguin et le point d'intérêt est un point d'une paroi du vaisseau sanguin.

Selon un aspect particulier de l'invention, la méthode est appliquée pour deux points d'intérêts correspondant à un premier point de la paroi distale du vaisseau sanguin et un second point de la paroi proximale du vaisseau sanguin, diamétralement opposé au premier point et la méthode comprend en outre une estimation du diamètre du vaisseau sanguin à partir des temps de vol extrapolés des deux points.

L'invention a pour objet un dispositif d'imagerie ultrasonore comprenant une sonde ultrasonore et une unité de traitement configurée pour réaliser les étapes de la méthode selon l'invention.

L'invention permet ainsi d'optimiser la précision des mesures sans nécessité de modifier la sonde ultrasonore.

D'autres caractéristiques et avantages de la présente invention apparaîtront mieux à la lecture de la description qui suit en relation aux dessins annexés suivants.
[Fig. 1] représente un schéma d'une sonde multiéléments ultrasonore apte à réaliser une séquence d'acquisition de signaux ultrasonores pour imager un vaisseau sanguin,
[Fig. 2] représente un organigramme des étapes d'une méthode de mesure d'un signal ultrasonore selon un mode de réalisation de l'invention,
[Fig. 3a] représente un exemple d'image ultrasonore obtenue pour caractériser un vaisseau sanguin,
[Fig. 3b] représente un signal ultrasonore extrait d'une colonne de l'image de la figure 3a,
[Fig. 4a] représente un schéma d'une sonde ultrasonore selon une première configuration de taille de fenêtre active en réception,
[Fig. 4b] représente un schéma d'une sonde ultrasonore selon une deuxième configuration de taille de fenêtre active en réception,
[Fig. 4c] représente un schéma d'une sonde ultrasonore selon une troisième configuration de taille de fenêtre active en réception,
[Fig. 5] représente un exemple de signal ultrasonore obtenu selon une configuration particulière de taille de fenêtre active,
[Fig. 6a] représente un diagramme donnant la variation du temps de vol d'un premier point d'intérêt du signal ultrasonore en fonction de la taille de fenêtre active,
[Fig. 6b] représente un diagramme donnant la variation du temps de vol d'un second point d'intérêt du signal ultrasonore en fonction de la taille de fenêtre active,
[Fig. 6c] représente un diagramme d'un modèle mathématique de la variation du diamètre estimé d'un vaisseau sanguin à partir des temps de vol des deux points d'intérêt mesurés aux figures 6a et 6b,

L'invention est à présent décrite dans le cadre d'application d'une imagerie médicale d'un vaisseau sanguin, par exemple une artère. Bien que l'invention s'applique avantageusement à cet exemple, elle n'est pas limitée à ce cas particulier et s'applique plus généralement pour tout type d'objet à imager, en particulier dans le domaine du contrôle non destructif et pour tout point d'intérêt d'un objet que l'on cherche à localiser de façon précise.

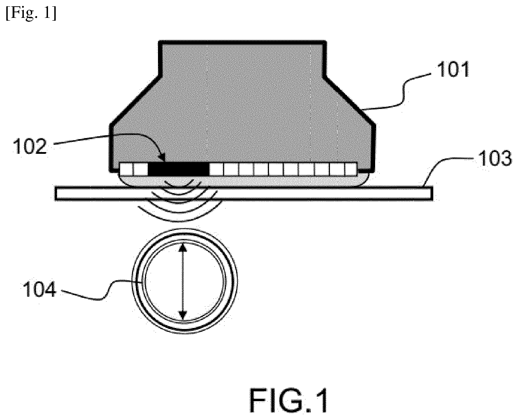

La figure 1 schématise un exemple de sonde ultrasonore multiéléments apte à imager une zone du corps humain à l'aide d'un faisceau ultrasonore. Sur l'exemple de la figure 1, la zone correspond à un plan transversal d'un vaisseau sanguin que l'on souhaite localiser précisément ou dont on souhaite déterminer avec précision le diamètre.

Dans l'exemple de la figure 1, la sonde 101 est positionnée au contact de la peau 103 de sorte que la rangée d'éléments 102 se trouve sensiblement dans un plan transversal d'une artère 104 à imager. La sonde 101 n'est pas nécessairement centrée sur l'artère 104, il suffit que l'artère soit couverte en émission et en réception par le faisceau ultrasonore généré par la sonde de sorte à obtenir une empreinte transversale de l'artère.

Pour réaliser une acquisition, la sonde est positionnée en un point donné et on active un groupe d'éléments en émission de sorte à générer un champ ultrasonore dans une direction donnée. Les échos du champ sur la zone couverte sont ensuite mesurés par le même groupe d'éléments ou un groupe d'éléments en nombre restreint. Dans l'exemple de la figure 1, le champ ultrasonore est émis dans une direction perpendiculaire à l'axe de l'alignement des éléments 102 qui correspond aussi localement au plan de la peau 103 sur laquelle est positionnée la sonde 101.

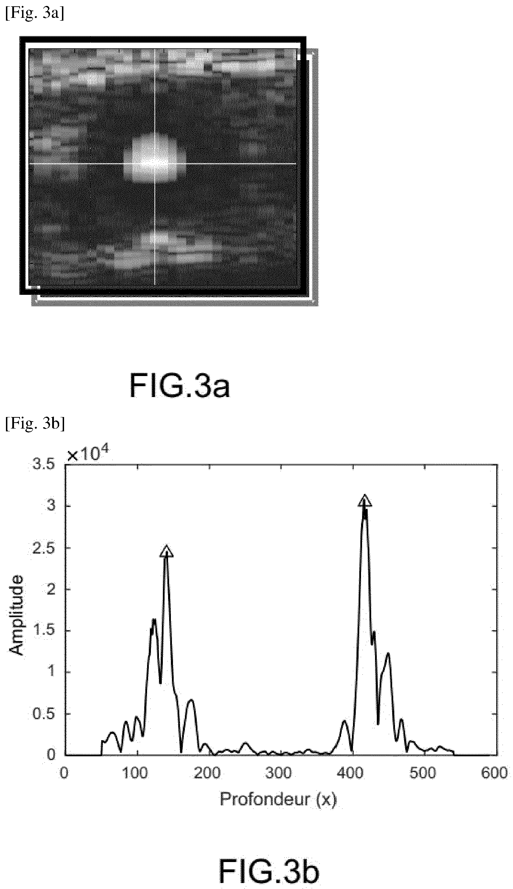

La figure 3a montre un exemple d'image 2D obtenue en réalisant plusieurs acquisitions successives en décalant à chaque fois le centre d'émission du champ ultrasonore par rapport à la zone couverte.

La figure 3b montre une colonne de l'image 2D de la figure 3a qui correspond à un signal mesuré pour une acquisition correspondant à une position de la sonde située à l'aplomb du centre du vaisseau à imager.

En effet, l'image 2D de la figure 3a correspond à une matrice dont chaque colonne est l'un des signaux ultrasonores acquis par la sonde pour une position donnée du champ émis par rapport à la zone imagée.

Sur la figure 3b, on a identifié par deux triangles, deux extrema qui correspondent aux échos du champ ultrasonore sur les parois distale et proximale du vaisseau sanguin (en l'occurrence une artère radiale).

Ces deux points correspondent à des points d'intérêt dans le contexte de la détermination du diamètre du vaisseau. En effet, à partir de la mesure des temps de vol de ces deux extrema, il est possible d'en déduire les positions des deux points et ensuite le diamètre en supposant que le signal correspond à un trajet qui passe par le centre de l'artère. Le diamètre interne du vaisseau est égal à D=0.5C.(t₂-t₁), où t₂, t₁ sont les abscisses des deux extrema (temps de vol) et C la vitesse de propagation de l'onde ultrasonore dans le milieu, en l'occurrence le sang.

Cependant, la précision de la mesure du temps de vol d'un point d'intérêt dépend de la taille de la fenêtre active de la sonde en émission comme cela est à présent explicité.

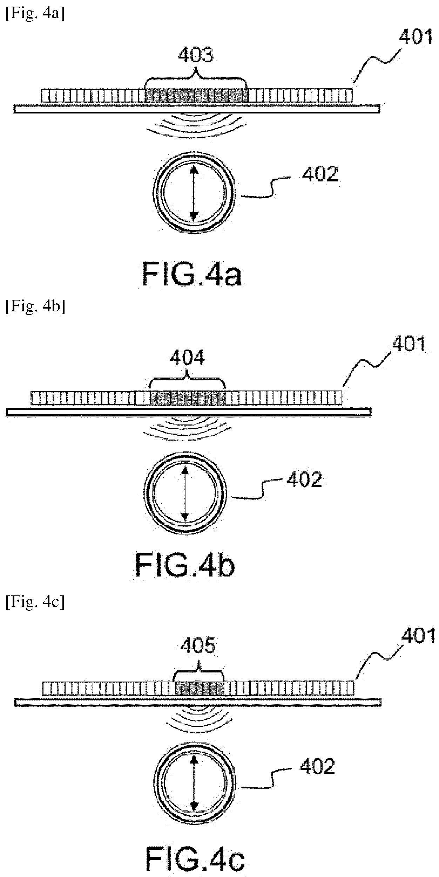

Les figures 4a, 4b et 4c montrent trois configurations d'acquisition différentes pour une même sonde ultrasonore 401 disposée à l'aplomb d'une artère radiale 402

Pour la première configuration représentée à la figure 4a, le nombre d'éléments actifs 403 en émission et en réception est égal à 16. Autrement dit, le champ ultrasonore est généré à partir des 16 éléments centraux de la sonde et mesuré également par ces 16 éléments. Cette acquisition permet d'obtenir une première mesure du signal représenté à la figure 3b.

La deuxième configuration schématisée à la figure 4b montre une situation où le nombre d'éléments actifs 404 en réception est égal à 12. Dans cette configuration, le nombre d'éléments actifs en émission peut être également pris égal à 12 ou peut être pris égal à 16 c'est-à-dire le nombre d'éléments actifs en émission maximal prédéfini selon l'application visée.

La troisième configuration représentée à la figure 4c montre encore une autre situation où le nombre d'éléments actifs 405 en réception est égal à 8. Le nombre d'éléments actifs en émission est égal à 8 ou 16.

De façon générale, les figures 4a,4b et 4c montrent qu'il est possible de réaliser la même mesure à partir d'une taille de fenêtre active variable. Plus la taille de fenêtre est grande, plus le rapport signal à bruit est élevé, mais moins la précision de mesure de l'écho en un point donné est importante. A l'inverse, une taille de fenêtre active minimum est nécessaire pour obtenir une mesure avec un rapport signal à bruit exploitable.

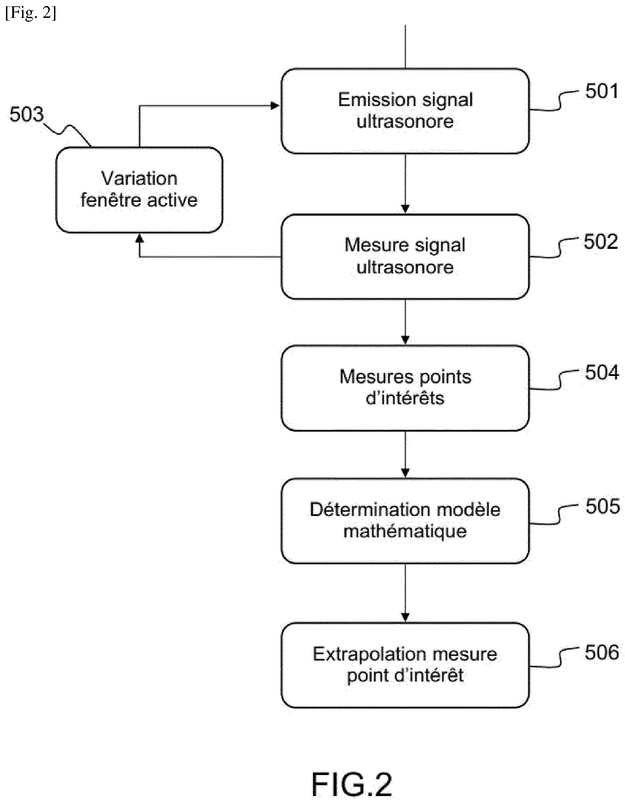

En référence à la figure 2, la méthode de mesure selon l'invention débute par les étapes 201,202,203 qui consistent à réaliser plusieurs acquisitions successives du même signal ultrasonore (pour la même position de la sonde par rapport à l'objet à imager) en faisant varier à chaque itération la taille de la fenêtre active.

Par exemple, dans le cas d'un transducteur multiéléments, le nombre d'éléments actifs en réception peut être réduit à chaque itération par rapport au nombre maximal d'éléments utilisés pour générer le faisceau ultrasonore. A chaque itération, le nombre d'éléments actifs en émission peut être laissé constant ou le groupe d'éléments actifs en émission peut être pris égal au groupe d'éléments actifs en réception.

Par exemple, à chaque itération, le nombre d'éléments actifs en réception peut être diminué d'un facteur 2.

Selon un autre mode de réalisation, si la sonde à ultrasons est fabriquée dans une technologie qui permet avec un seul élément de faire varier l'ouverture du champ ultrasonore, alors la variation du nombre d'éléments actifs en réception est remplacée par une variation du champ d'ouverture à chaque itération.

A l'issue de l'ensemble des itérations des étapes 201,202,203, on obtient N mesures du même signal ultrasonore, avec N le nombre d'acquisitions réalisées.

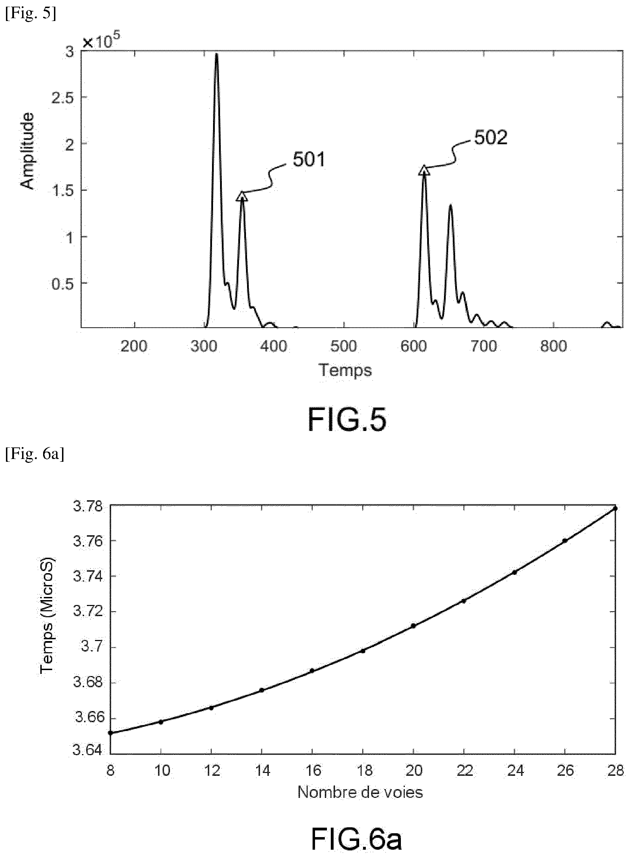

On identifie ensuite un ou plusieurs points d'intérêt sur le signal ultrasonore acquis. Par exemple, pour le cas du signal de la figure 5, deux points d'intérêt 501,502 sont identifiés qui correspondent à des échos du signal sur les parois distale et proximale internes du vaisseau sanguin.

On souhaite déterminer avec précision, pour chacun de ces deux points, le temps de vol ou la profondeur associée (les deux grandeurs étant liées entre elles par la vitesse de propagation de l'onde ultrasonore dans le milieu situé entre la sonde et l'artère) qui est représentée en abscisse du diagramme de la figure 5.

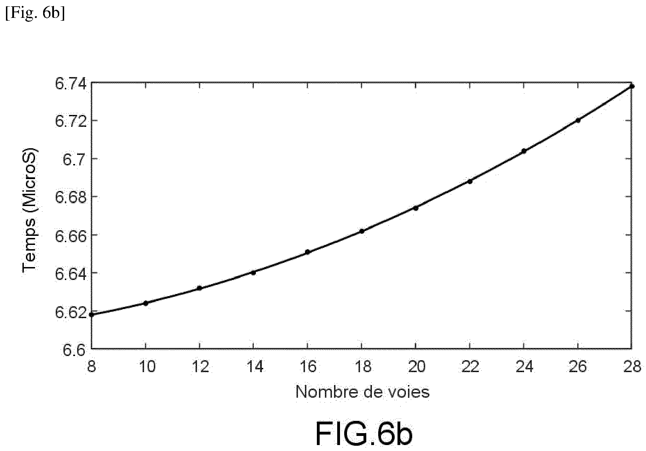

Pour chaque point d'intérêt considéré, on relève son abscisse (exprimée en temps de vol ou en distance) et on trace (à l'étape 204) les diagrammes représentés aux figures 6a et 6b qui donnent l'évolution de ces mesures en fonction du nombre d'éléments actifs en réception encore appelé nombre de voies sur les figures 6a et 6b. La figure 6a correspond à la position de l'écho du signal sur la paroi proximale et la figure 6b correspond à la position de l'écho du signal sur la paroi distale. Sur les exemples illustrés, le nombre de voies varie de 8 à 28, mais cet exemple n'est pas limitatif. En ordonnée, on retrouve le temps de vol de chaque mesure.

A l'étape 205, on détermine ensuite, par optimisation mathématique, un modèle numérique de l'évolution des courbes de variation du temps de vol en fonction du nombre de voies pour chaque point d'intérêt.

Ce modèle mathématique est par exemple déterminé par un modèle sous forme d'une fonction polynomiale du second degré ou tout autre modèle numérique adapté. Il peut être défini au moyen d'une interpolation des points de la courbe mesurés. Cette interpolation peut être réalisée au moyen d'un mélange de fonctions Gaussiennes ou apprise par un modèle entrainé de réseaux de neurones ou toute autre algorithme d'optimisation numérique adapté.

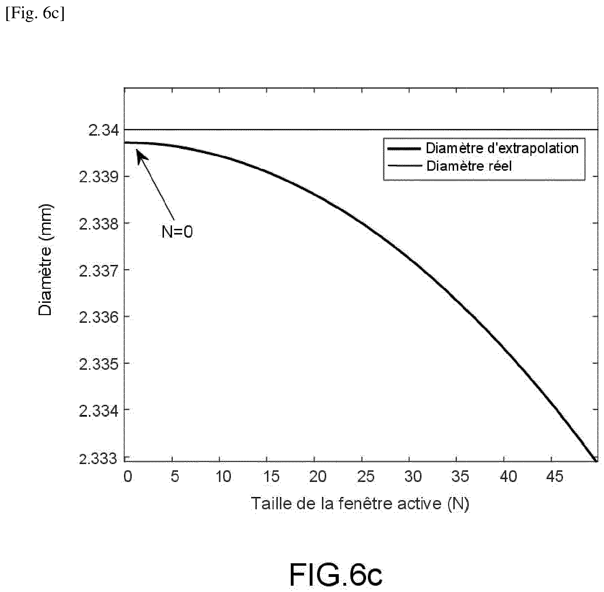

Sur la figure 6c on a représenté un exemple d'un tel modèle numérique déterminé pour la différence entre les deux courbes des figures 6a et 6b qui correspond, dans le cas d'application visé, au diamètre du vaisseau sanguin.

Le modèle mathématique peut être déterminé pour chaque courbe associée à chaque point d'intérêt ou pour la grandeur finale que l'on souhaite déterminer à partir de plusieurs points d'intérêt, en l'espèce ici la différence entre les deux temps de vol.

Enfin à l'étape 206, le modèle mathématique obtenu est extrapolé pour déterminer une valeur asymptotique pour un nombre de voies tendant vers 0. En effet, cette valeur correspond à la mesure la plus précise en termes de résolution et sa détermination via une extrapolation d'un modèle permet de ne pas être soumis au problème de faible rapport signal à bruit inhérent à une mesure réalisée avec une fenêtre active très petite.

Le diamètre du vaisseau est par exemple déterminé à l'aide de la relation suivante :

D=0.5(t_{distal}(0)- tₚᵣₒₓᵢₘₐₗ(0)).C, où C désigne la vitesse de propagation de l'onde ultrasonore dans le milieu traversé, t_{distal}(0) et tₚᵣₒₓᵢₘₐₗ(0) sont les valeurs extrapolées pour un nombre de voies tendant vers 0 des modèles d'évolution des temps de vol associés aux points d'intérêt correspondant aux échos du champ ultrasonore respectivement sur la paroi distale et la paroi proximale du vaisseau.

De manière générale, l'invention peut s'appliquer à l'identique pour améliorer la précision d'une mesure de temps de vol d'un point d'intérêt correspondant à l'écho d'un champ ultrasonore sur la surface d'un objet à imager.

### Références

[1] J. H. Gagan et al., "Automated Segmentation of Common Carotid Artery in Ultrasound Images," in IEEE Access, vol. 10, pp. 58419-58430, 2022
[2] « System A feasibility study of a PMUT-based wearable sensor for the automatic monitoring of carotid artery parameters », 2021 IEEE International Ultrasonics Symposium (IUS)

## Revendications

1. Méthode de caractérisation d'un objet au moyen d'une sonde ultrasonore, la méthode comprenant les étapes de :
- Réaliser plusieurs itérations des étapes de :
i. Emettre (201) un champ ultrasonore ayant une ouverture d'une taille donnée,
ii. Mesurer (202) un écho du champ ultrasonore après réflexion sur une zone d'intérêt d'un objet à imager,
iii. A chaque nouvelle itération, modifier (203) la taille de l'ouverture du champ ultrasonore,
- Pour au moins un point d'intérêt de la zone d'intérêt, relever (204), sur la mesure de l'écho, le temps de vol associé,
- Déterminer (205), à partir desdites mesures de temps de vol, un modèle de variation des temps de vol en fonction de la taille de l'ouverture du champ ultrasonore,
- Extrapoler (206) le temps de vol du point d'intérêt pour une taille d'ouverture tendant vers 0 à partir dudit modèle.

2. Méthode de caractérisation d'un objet selon la revendication 1 dans laquelle la sonde ultrasonore (101) comprend une pluralité d'éléments (102) et la variation de la taille de l'ouverture du champ ultrasonore est obtenue en variant le nombre d'éléments actifs en réception.

3. Méthode de caractérisation d'un objet selon la revendication 2 dans laquelle le nombre d'éléments actifs en réception est un multiple de deux.

4. Méthode de caractérisation d'un objet selon l'une quelconque des revendications 2 ou 3 dans laquelle le nombre d'éléments actifs en émission est égal au nombre d'éléments actifs en réception ou au nombre maximal d'éléments que comprend la sonde.

5. Méthode de caractérisation d'un objet selon l'une quelconque des revendications précédentes dans laquelle l'objet à imager est un vaisseau sanguin et le point d'intérêt est un point d'une paroi du vaisseau sanguin.

6. Méthode de caractérisation d'un objet selon la revendication 5 dans laquelle la méthode est appliquée pour deux points d'intérêts correspondant à un premier point de la paroi distale du vaisseau sanguin et un second point de la paroi proximale du vaisseau sanguin, diamétralement opposé au premier point et la méthode comprend en outre une estimation du diamètre du vaisseau sanguin à partir des temps de vol extrapolés des deux points.

7. Dispositif d'imagerie ultrasonore comprenant une sonde ultrasonore et une unité de traitement configurée pour réaliser les étapes de la méthode selon l'une quelconque des revendications précédentes.

## Patentansprüche

1. Verfahren zur Charakterisierung eines Objekts mittels einer Ultraschallsonde, wobei das Verfahren die folgenden Schritte umfasst:
- Durchführen mehrerer Iterationen der folgenden Schritte:
i. Emittieren (201) eines Ultraschallfeldes, das eine Öffnung einer gegebenen Größe aufweist,
ii. Messen (202) eines Echos des Ultraschallfeldes nach Reflexion an einem Bereich von Interesse eines abzubildenden Objekts,
iii. bei jeder neuen Iteration, Modifizieren (203) der Größe der Öffnung des Ultraschallfeldes,
- für mindestens einen Punkt von Interesse des Bereichs von Interesse, Feststellen (204), an der Echomessung, der assoziierten Flugzeit,
- Bestimmen (205), ausgehend von den Flugzeitmessungen, eines Modells zur Variation der Flugzeit in Funktion der Größe der Öffnung des Ultraschallfeldes,
- Extrapolieren (206) der Flugzeit des Punkts von Interesse für eine Öffnungsgröße, die nach 0 tendiert, ausgehend von dem Modell.

2. Verfahren zur Charakterisierung eines Objekts nach Anspruch 1, wobei die Ultraschallsonde (101) eine Vielzahl von Elementen (102) umfasst und die Variation der Größe der Öffnung des Ultraschallfeldes durch Variieren der Anzahl an aktiven Elementen beim Empfang erhalten wird.

3. Verfahren zur Charakterisierung eines Objekts nach Anspruch 2, wobei die Anzahl an aktiven Elementen beim Empfang ein Vielfaches von zwei ist.

4. Verfahren zur Charakterisierung eines Objekts nach einem der Ansprüche 2 oder 3, wobei die Anzahl an aktiven Elementen bei der Emission gleich der Anzahl an aktiven Elementen beim Empfang oder der maximalen Anzahl an Elementen ist, die die Sonde umfasst.

5. Verfahren zur Charakterisierung eines Objekts nach einem der vorstehenden Ansprüche, wobei das abzubildende Objekt ein Blutgefäß ist und der Punkt von Interesse ein Punkt einer Wand des Blutgefäßes ist.

6. Verfahren zur Charakterisierung eines Objekts nach Anspruch 5, wobei das Verfahren für zwei Punkte von Interesse angewendet wird, die einem ersten Punkt der distalen Wand des Blutgefäßes und einem zweiten Punkt der proximalen Wand des Blutgefäßes entsprechen, der dem ersten Punkt diametral gegenüberliegt, und wobei das Verfahren weiter eine Abschätzung des Durchmessers des Blutgefäßes ausgehend von den von den zwei Punkten extrapolierten Flugzeiten umfasst.

7. Ultraschallbildgebungsvorrichtung, umfassend eine Ultraschallsonde und eine Behandlungseinheit, die konfiguriert ist zum Durchführen der Schritte des Verfahrens nach einem der vorstehenden Ansprüche.

## Claims

1. Method for characterising an object using an ultrasound probe, the method comprising the following steps of:
- carrying out several iterations of the following steps of:
i. transmitting (201) an ultrasound field having an aperture with a given size;
ii. measuring (202) an echo of the ultrasound field following reflection on a zone of interest of an object to be imaged;
iii. modifying (203), for each new iteration, the size of the aperture of the ultrasound field;
- recording (204), for at least one point of interest in the zone of interest, the associated time-of-flight on the measurement of the echo;
- determining (205), based on said time-of-flight measurements, a variation model of the times-of-flight as a function of the size of the aperture of the ultrasound field;
- extrapolating (206) the time-of-flight of the point of interest for an aperture size tending towards 0 based on said model.

2. Method for characterising an object according to Claim 1, wherein the ultrasound probe (101) comprises a plurality of elements (102) and the variation in the size of the aperture of the ultrasound field is obtained by varying the number of reception active elements.

3. Method for characterising an object according to Claim 2, wherein the number of reception active elements is a multiple of two.

4. Method for characterising an object according to any one of Claims 2 or 3, wherein the number of transmission active elements is equal to the number of reception active elements or to the maximum number of elements included in the probe.

5. Method for characterising an object according to any one of the preceding claims, wherein the object to be imaged is a blood vessel and the point of interest is a point on a wall of the blood vessel.

6. Method for characterising an object according to Claim 5, wherein the method is applied for two points of interest corresponding to a first point on the distal wall of the blood vessel and a second point on the proximal wall of the blood vessel, diametrically opposite the first point, and the method further comprises estimating the diameter of the blood vessel based on the extrapolated times-of-flight of the two points.

7. Ultrasound imaging device comprising an ultrasound probe and a processing unit configured to carry out the steps of the method according to any one of the preceding claims.
